# EUROPÄISCHE PATENTSCHRIFT

(11) **EP 0 409 001 B1**
(45) Veröffentlichungstag und Bekanntmachung des Hinweises auf die Patenterteilung: **10.11.1993**
(21) Anmeldenummer: 90112837.1
(22) Anmeldetag: 05.07.1990
(51) Int. Cl.: F04B 43/08

(54) **Vorrichtung zum Injizieren von Flüssigkeiten**
Liquid injection device
Dispositif d'injection de liquides

(30) Priorität: 15.07.1989 DE 3923457
(43) Veröffentlichungstag der Anmeldung: 23.01.1991
(73) Patentinhaber: Fresenius AG, 61350 Bad Homburg (DE)
(72) Erfinder: Faeser, Ulrich, Dipl.-Ing., D-6242 Kronberg 2 (DE)
(74) Vertreter: Fuchs Mehler Weiss

(56) Entgegenhaltungen:
- EP-A- 0 090 440
- EP-A- 0 319 276
- DE-C- 362 284
- FR-A- 2 492 902

## Beschreibung

Die Erfindung betrifft eine Vorrichtung zum Injizieren von Flüssigkeiten nach dem Oberbegriff des Anspruches 1.

Vorrichtungen zum Injizieren von Flüssigkeiten in Form von Infusionspumpen werden als Schlauchpumpen oder als Pumpen ohne Schlauch mit besonderer Förderkammer ausgeführt. In jedem Falle ist das stetig förderbare Flüssigkeitsvolumen, verfahrenstechnisch bedingt, begrenzt. Die Begrenzung ergibt sich bei Schlauchpumpen auch aus der Länge der Förderstrecke und bei Pumpen mit besonderer Förderkammer aus dem Volumen derselben. Es ist damit neben oder nach der Förderung des begrenzten Volumens ein Nachfüllen dieses Volumens notwendig.

Soll eine Förderung einer Flüssigkeit mit konstanter Fließgeschwindigkeit erfolgen, muß ein konstanter Druck im patientenseitigen Teil des Überleitgerätes aufrecht erhalten werden.

Das nachgefüllte Volumen steht unter hydrostatischem Druck, der durch die Höhendifferenz zwischen Flüssigkeitsreservoir und Förderelement bestimmt ist. Dieser hydrostatische Druck ist bei bestimmungsgemäßer Anwendung einer Infusionspumpe immer kleiner als der Flüssigkeitsdruck in Förderrichtung, also im patientenseitigen Teil des Überleitgerätes.

Eine Vorrichtung zum Injizieren von Flüssigkeit der im Oberbegriff des Anspruchs 1 angegebenen Art ist beispielsweise aus der EP A 0 024 431 bekannt. Bei dieser Vorrichtung wird eine speziell ausgebildete Pumpenkammer verwendet, die als Einweg-Pumpenkammer ausgebildet ist und die zwei sog. flexible Rollmembran-Pumpenkammern aufweist, die im unbelasteten Zustand ein vorbestimmtes Volumen begrenzen, das während des Pumpvorganges von entsprechend geformten Pumpstößeln vermindert wird. Um die gewünschte Funktionsweise zu erreichen, weist die gattungsgemäße Vorrichtung zwei Pumpenstößel sowie ein Einlaßventil und ein Auslaßventil auf. Ferner ist ein dritter Ventilkolben erforderlich, der nicht angetrieben ist, sondern sich in Abhängigkeit von einem Fluiddruck in einer Druckerfassungskammer der Pumpenkammer bewegt.

Unterstellt man, daß die Forderung nach konstanter Flußgeschwindigkeit über das verfahrenstechnisch bedingte endliche Fördervolumen erfüllt ist, ergeben sich jedoch bei der gattungsgemäßen Vorrichtung Probleme beim Übergang auf das nächste Fördervolumen. Dieser kritische Zeitpunkt ist immer dann erreicht, wenn das Einlaßventil der gattungsgemäßen Vorrichtung geschlossen werden muß, damit das Auslaßventil geöffnet werden kann. Bei der gattungsgemäßen Vorrichtung ist der Übergang von einer Förderphase zur nächsten Förderphase daher mit einem temporären Druckabfall im patientenseitigen Teil des Überleitgerätes verbunden, was eine Verminderung der Strömungsgeschwindigkeit, also eine Verminderung des Förderungsvolumens pro Zeiteinheit, zur Folge hat. Dies wiederum reduziert die Genauigkeit der Förderrate insbesondere bei kleinen Werten und bringt bei kleinen Meßzeiten der Förderrate hohe Abweichungen mit sich. Dies bedeutet mit anderen Worten, daß kleine Förderraten über einen langen Zeitraum aufrecht erhalten werden müssen, damit überhaupt mit einer akzeptierbaren Genauigkeit das in der Zeiteinheit geförderte Sollvolumen erreicht wird.

Eine weitere Pumpe, die derjenigen der EP-A-24431 gleicht, ist in dem GB-Patent 2065789 beschrieben. Insofern treten dort auch ahnliche Probleme auf.

Ferner zeigt DE-C-362 284 eine Balgpumpe für Gasförderung, welche mit fünf Hebeln statt mit vier Stößeln ausgestattet ist.

Schließlich offenbart die FR-A-2 492 902 eine Vorrichtung, bei der jeder Stößel einerseits als Ventil wirkt und andererseits auch Förderfunktion hat.

Es ist daher Aufgabe der vorliegenden Erfindung, eine Vorrichtung zum Injizieren von Flüssigkeiten der im Oberbegriff des Anspruches 1 angegebenen Art zu schaffen, die eine äußerst gleichmäßige Förderung des Mediums bei gleichzeitig geringem apparativem Aufwand ermöglicht.

Die Lösung dieser Aufgabe erfolgt durch die Merkmale des Anspruches 1.

Durch die erfindungsgemäße Vorrichtung wird ein Fördermechanismus einer peristaltikähnlichen Schlauchpumpe geschaffen, der insgesamt lediglich vier Stößel benötigt, die den Schlauch gegen die Druckplatte im Querschnitt verjüngen können, soweit bis ein Verschluß des Querschnittes erreicht ist. Hierbei richtet sich der Schlauch durch die schlaucheigenen Rückstellkräfte bis auf seinen vollen Querschnitt auf, wenn sich die Stößel von der Druckplatte wegbewegen.

Zu den besonderen Vorteilen der erfindungsgemäßen Vorrichtung zählt ein äußerst gleichmäßiges Fördervolumen pro Zeit, wobei aufgrund der Tatsache, daß das Auslaßventil als Pumpenstößel ausgebildet ist, ein Wegfall von Ventilwechselzeiten erreicht wird. Dies vergleichmäßigt den Fluß des zu pumpenden Mediums, ohne daß hierfür ein hoher apparativer Aufwand betrieben werden muß.

Es ergeben sich mithin bei der Betätigung des Fördermechanismus drei Förderkammern, wobei lediglich der einlaßseitige Stößel ausschließlich Ventilfunktion hat.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung eines Ausführungsbeispiels anhand der Zeichnung.

Es zeigt:
- Fig. 1: eine schematische, stark vereinfachte Zeichnung einer möglichen Ausführungsform einer erfindungsgemäßen Vorrichtung,
- Fig. 2: eine schematische Darstellung des Arbeitszyklus der Vorrichtung gemäß Figur 1,
- Fig. 3: eine Prinzipskizze der Lage der Stößel der erfindungsgemäßen Vorrichtung jeweils am Beginn einer Phase und
- Fig. 4: einen der Fig. 3 zugeodneten Phasenplan der Stößel der erfindungsgemäßen Vorrichtung.

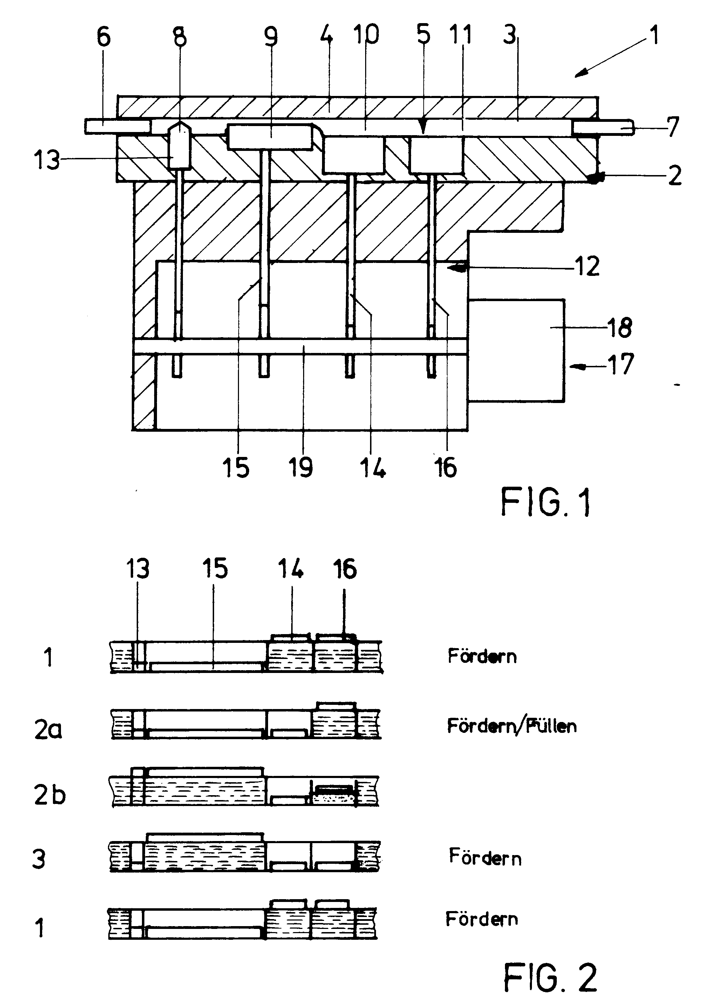

In Figur 1 ist eine schematisch stark vereinfachte Darstellung einer erfindungsgemäßen Vorrichtung 1 zum Injizieren von Flüssigkeit wiedergegeben, die ein Gehäuse 2 mit einem Aufnahmeraum 3 und einer Druckplatte 4 aufweist. Die erfindungsgemäße Vorrichtung 1 weist ferner einen elastischen Förderleitungsabschnitt 5 auf, der im Aufnahmeraum 3 angeordnet ist und der einen mit einer nicht näher dargestellten Zuführleitung verbindbaren Einlaß 6 sowie einen mit einer ebenfalls nicht näher dargestellten Abführleitung verbindbaren Auslaß 7 aufweist. Im Förderleitungsabschnitt 5 können ein Absperrabschnitt 8 sowie Förderkammern 9 bis 11 in noch näher zu beschreibender Art und Weise ausgebildet werden.

Hierfür weist die erfindungsgemäße Vorrichtung 1 eine Fördereinrichtung 12 auf, die ein Einlaßventil 13 umfaßt, das in Förderrichtung gesehen stromab des Einlasses 6 angeordnet ist. Ferner ist ein Auslaßventil 14 stromauf des Auslasses 7 angeordnet. Dieses Auslaßventil 14 ist jedoch im Gegensatz zu bisher bekannten Vorrichtungen als Pumpenstößel ausgebildet, so daß die erfindungsgemäße Vorrichtung 1 zusammen mit einem weiteren ersten und zweiten Pumpenstößel 15 bzw. 16 insgesamt 3 Pumpenstößel aufweist, die die Förderkammern 9 bis 11 zyklisch vergrößern und verkleinern, wobei der erste Pumpenstößel 15 zwischen dem Einlaßventil 13 und dem Auslaßventil bzw. Pumpenstößel 14 und der zweite Pumpenstößel 16 zwischen dem Auslaßventil bzw. Pumpenstößel 14 und dem Auslaß 7 angeordnet ist.

Zu den besonderen Vorteilen der erfindungsgemäßen Vorrichtung 1 zählt, daß der Förderleitungsabschnitt 5 als ein einfaches Schlauchsegment ausgebildet ist, das im durch die Pumpenstößel und das Einlaßventil unbeaufschlagten Zustand einen durchgehend gleichen Durchmesser aufweist.

Die Förderkammern 9 bis 11 der erfindungsgemäßen Vorrichtung werden durch die Wirkung der Pumpenstößel 14 bis 16 gebildet, die das Schlauchsegment 5 zusammendrücken, wobei beim Zurückbewegen dar Pumpenstößel weg von der Druckplatte 4 die somit gebildeten Pumpenkammern aufgrund der schlaucheigenen Rückstellkräfte wieder bis zum ursprünglichen Querschnitt des Schlauchsegmentes 5 vergrößert werden.

Die Bewegung der Pumpenstößel 14 bis 16 sowie des Einlaßventiles 13 wird durch eine Antriebseinrichtung 17 bewirkt, die in üblicher Art und Weise einen Motor 18 mit einer Antriebswelle 19 aufweisen kann, auf der entsprechend der zu erzeugenden Bewegung und zeitlichen Abfolge der Bewegung der Stößel geeignet ausgebildete Nocken angeordnet sein können.

Zur Erläuterung der Funktionsweise der erfindungsgemäßen Vorrichtung 1, die sich unmittelbar aus der Betrachtung der Figuren 2 bis 4 erschließt, wird beispielhaft von folgenden Voraussetzungen ausgegangen:

Es wird davon ausgegangen, daß die möglichen Bewegungsgeschwindigkeiten der Stößel 13 bis 16 V1 und V2 sind. Die Geschwindigkeit V1 ist dem Stößel bzw. Einlaßventil 13 zugeordnet, während sich Stößel 15 mit V2 schließt und mit V1 öffnet. Die Stößel 14 und 16 öffnen und schließen sich jeweils mit V2, wobei V1 größer sei als V2.

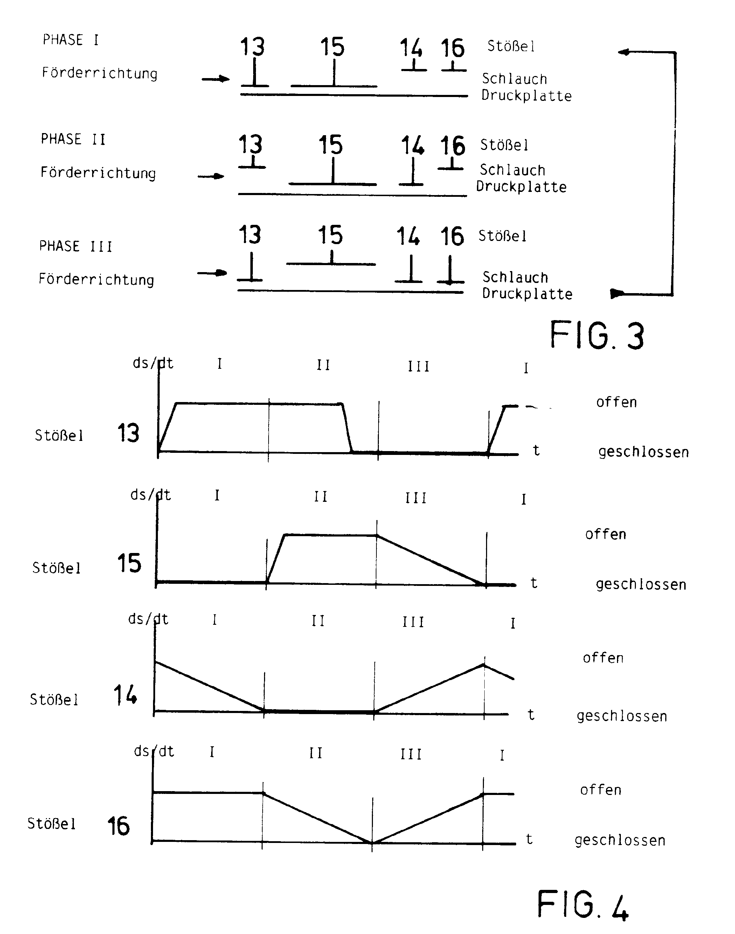

Unter diesen Voraussetzungen sind die Phasenbeziehungen gemäß dem in Figur 4 dargestellten Phasenplan eingehalten.

Ferner ist bezüglich der Geometrie der Stößel 14 und 16 zu sagen, daß sie die Länge l aufweisen, während der Stößel 15 die Länge 3 l aufweist. Da der Stößel 13 als Einlaßventil ausgebildet ist, hat seine Länge im Prinzip keinen Einfluß auf die Funktionsweise.

Wie sich aus den Figuren 3 und 4 erschließt, bleibt der Stößel 15 in Phase I geschlossen. Stößel 13 öffnet sich mit V1 und Stößel 14 bewegt sich mit V2 bis zum Schaluchverschluß in Richtung Druckplatte 4, während Stößel 16 offen bleibt. Es wird das unter Stößel 14 befindliche Volumen gefördert.

Mit Phase II beginnt die Förderung des unter dem Stößel 16 befindlichen Volumenelementes durch Schließen mit V2. Während dieser Phase wird Stößel 15 mit V1 geöffnet und Stößel 13 mit V1 geschlossen. Hieraus ergibt sich die Füllung des unter Stößel 15 befindlichen Schlauchvolumens.

In Phase III bewegen sich die Stößel 15, 14 und 16 gleichzeitig und mit gleichen Geschwindigkeitsbeträgen V2. Stößel 15 bewegt sich gegen die Druckplatte 4 und Stößel 14 und 16 bewegen sich gemeinsam von der Druckplatte 4 weg. Stößel 13 bleibt geschlossen. Damit wird wegen der Längenverhältnisse Stößel 15 zu Stößel 14 bzw. Stößel 15 zu Stößel 16 wieder ein Volumen entsprechend unter dem Stößel 14 bzw. Stößel 16 liegenden Volumen gefördert.

Alle Phasen I bis III sind im patientenseitigen Teil (Druckteil) des Überleitgerätes Förderphasen, wobei während der Phase I die Füllung vorbereitet und in Phase II das Füllen der in den Phasen I bis III geförderten Volumen im Druckteil unter gleichzeitiger Förderung wie während der Phasen I und III geschieht. Dadurch wird der zuvor anhand der gattungsgemäßen Vorrichtung beschriebene Druckabfall ausgeschlossen, sofern die Bedingungen eingehalten werden.

Der zuvor beschriebene Phasenablauf gemäß Figuren 3 und 4 entspricht den in Fig. 2 angegebenen Funktionen der erfindungsgemäßen Vorrichtung in den Schritten 1, 2a, 2b, 3 und 1.

## Patentansprüche

1. Vorrichtung (1) zum Injizieren von Flüssigkeiten mit einem Gehäuse (2), das einen Aufnahmeraum (3) und eine Druckplatte (4) aufweist;
mit einem elastischen Förderleitungsabschnitt (5), der im Aufnahmeraum (3) angeordnet ist, der einen mit einer Zuführleitung verbindbaren Einlaß (6) aufweist, der einen mit einer Abführleitung verbindbaren Auslaß (7) aufweist, und in dem Förderkammern (9 - 11) ausbildbar sind;
mit einer Fördereinrichtung (12), die ein Einlaßventil (13) in Förderrichtung gesehen stromab des Einlasses (6) aufweist, die ein Auslaßventil (14) stromauf des Auslasses (7) aufweist, und die einen ersten und einen zweiten Pumpenstößel (15 bzw. 16) aufweist, die die Förderkammern (9 - 11) zyklisch vergrößern und verkleinern, wobei der erste Pumpenstößel (15) zwischen dem Einlaßventil (13) und dem Auslaßventil (14) und dem Auslaß (7) angeordnet ist,
dadurch gekennzeichnet, daß
der Förderleitungsabschnitt (5) als Schlauchsegment ausgebildet ist, das im unbeaufschlagten Zustand einen durchgehend gleichen Durchmesser aufweist,
wobei die Förderkammern (9 - 11) durch Zusammendrücken des Schlauchsegmentes (15) mittels der Pumpenstößel (14 - 16) und durch danach erfolgendes elastisches Ausdehnen aufgrund der schlaucheigenen Rückstellkräfte bis zum ursprünglichen Querschnitt gebildet werden;
daß das Auslaßventil (14) als weiterer Pumpenstößel ausgebildet ist, der bis zum vollständigen Schließen des entsprechenden Schlauchquerschnittes Förderfunktion hat;
und daß der zweite Pumpenstößel (16) sowie das als Pumpenstößel ausgebildete Auslaßventil (14) die gleiche sich in Schlauchrichtung erstreckende Länge ihrer Auflagefläche haben, während der erste Pumpenstößel (15) eine dreifach größere Länge (3) aufweist.

2. Vorrichtung nach Anspruch 1, dadurch gekennzeichnet, daß die Pumpenstößel (14 - 16) und das Einlaßventil (13) zwei unterschiedliche mögliche Bewegungsgeschwindigkeiten V1, V2, haben, wobei die Geschwindigkeit V1 größer ist als die Geschwindigkeit V2.

3. Vorrichtung nach Anspruch 2, dadurch gekennzeichnet, daß die Geschwindigkeit V1 dem Einlaßventil (13) zugeordnet ist.

4. Vorrichtung nach Anspruch 2 oder 3, dadurch gekennzeichnet, daß der erste Pumpenstößel (15) mit der Geschwindigkeit V2 schließt und mit der anderen Geschwindigkeit V1 öffnet.

5. Vorrichtung nach einem der Ansprüche 2 bis 4, dadurch gekennzeichnet, daß sich das Auslaßventil (14) und der zweite Pumpenstößel (16) mit der Geschwindigkeit V2 öffnen und schließen.

## Claims

1. Apparatus (1) for injecting fluid comprising a housing (2) having an accommodation space (3) and a pressure plate (4);
an elastic delivery conduit portion (5) which is arranged in the accommodation space (3), which has an inlet (6) connectable to a feed conduit, which has an outlet (7) connectable to a discharge conduit and in which conveying chamber (9-11) can be formed;
a delivery means (12) which has an inlet valve (13) downstream of the inlet (6) seen in the conveying direction, which has an outlet valve (14) upstream of the outlet (7) and which has a first and a second pump ram (15, resp. 16) which cyclically enlarge and diminish the delivery chambers (9-11), the first pump ram (15) being arranged between the inlet valve (13) and the outlet valve (14) and the outlet (7),
characterized in
that the delivery conduit portion (5) is formed as hose segment which in the uninfluenced state has a constant diameter throughout, with the delivery chambers (9-11) being formed by compression of the hose segment (15) by means of the pump rams (14-16) and by the expansion subsequently taking place due to the restoring forces inherent in the hose up to the original cross-section;
that the outlet valve (14) is formed as further pump ram which has a delivery function up to complete closure of the corresponding hose cross-section;
and that the second pump ram (16) and the outlet valve (14) constructed as pump ram have the same length of their support surface extending in hose direction whilst the first pump ram (15) has three times that length (3).

2. Apparatus according to claim 1, characterized in that the pump rams (14-16) and the inlet valve (13) have two different possible movement velocities V1, V2, with the velocity V1 being greater than the velocity V2.

3. Apparatus according to claim 2, characterized in that the velocity V1 is associated with the inlet valve (13).

4. Apparatus according to claim 2 or 3, characterized in that the first pump ram (15) closes with the velocity V2 and opens with the other velocity V1.

5. Apparatus according to any one of claims 2 to 4, characterized in that the outlet valve (14) and the pump ram (16) open and close with the velocity V2.

## Revendications

1. Dispositif (1) pour injecter des liquides, comportant un boîtier (2), qui comporte une chambre de logement (3) et une plaque de serrage (4);
un élément élastique (5) de conduite de refoulement, qui est disposé dans la chambre de logement (3) et qui possède une entrée (6) pouvant être raccordée à une conduite d'arrivée et comporte une sortie (7) pouvant être raccordée à une conduite d'évacuation, l'entrée et la sortie pouvant être réalisées dans les chambres de refoulement (9-11);
un dispositif de refoulement (12), qui comprend une soupape d'entrée (13) située en aval de l'entrée (6), lorsqu'on regarde dans la direction de refoulement, et possède une soupape de sortie (14) située en amont de la sortie (7), ces soupapes comportant des premier et second poussoirs de pompe (15 et 16), qui augmentent et réduisent cycliquement le volume des chambres de refoulement (9-11), le premier poussoir de pompe (15) étant disposé entre la soupape d'entrée (13) et la soupape de sortie (14) et la sortie (7),
caractérisé en ce que l'élément (5) de conduite de refoulement est agencé sous la forme d'un segment de tuyau, qui, à l'état non chargé, possède un diamètre constant sur toute sa longueur,
les chambres de refoulement (9-11) étant formées par compression du segment de tuyau (15) au moyen du poussoir de pompe (14-16) et par dilatation élastique ultérieure sur la base des forces de rappel, propres au tuyau, jusqu'à ce que la section transversale initiale soit atteinte; et
que la soupape de sortie (14) est agencée sous la forme d'un autre poussoir de pompe, qui possède une fonction de refoulement jusqu'à la fermeture complète de la section transversale correspondante du tuyau; et
que les surfaces d'application du second poussoir de pompe (16) ainsi que de la soupape de sortie (14) agencée sous la forme d'un poussoir de pompe possèdent la même longueur dans la direction du tuyau, tandis que le premier poussoir de pompe (15) possède une longueur (3) trois fois supérieure.

2. Dispositif selon la revendication 1, caractérisé en ce que les poussoirs de pompe (14-16) et la soupape d'entrée (13) possèdent deux vitesses de déplacement possibles différentes V1, V2, la vitesse V1 étant supérieure à la vitesse V2.

3. Dispositif selon la revendication 2, caractérisé en ce que la vitesse V1 est associée à la soupape d'entrée (13).

4. Dispositif selon la revendication 2 ou 3, caractérisé en ce que le premier poussoir de pompe (15) se ferme à la vitesse V2 et s'ouvre à l'autre vitesse V1.

5. Dispositif selon l'une des revendications 2 à 4, caractérisé en ce que la soupape de sortie (14) et le second poussoir de pompe (16) s'ouvrent et se ferment à la vitesse V2.
